# EUROPÄISCHE PATENTSCHRIFT

(11) **EP 0 736 507 B1**
(45) Veröffentlichungstag und Bekanntmachung des Hinweises auf die Patenterteilung: **06.05.1999**
(21) Anmeldenummer: 96101857.9
(22) Anmeldetag: 09.02.1996
(51) Int. Cl.: C07C 2/68, C07C 6/12

(54) **Verfahren zur Alkylierung und Transalkylierung von Aromaten**
Process for the alkylation and transalkylation of aromatic compounds
Procédé d'alkylation et transalkylation de composés aromatiques

(30) Priorität: 07.04.1995 DE 19513117
(43) Veröffentlichungstag der Anmeldung: 09.10.1996
(73) Patentinhaber: HÜLS AKTIENGESELLSCHAFT, 45764 Marl (DE)
(72) Erfinder: Heinrich, Lothar, Dr., D-48165 Münster (DE); Rauhut, Otto, D-45721 Haltern (DE)

(56) Entgegenhaltungen:
- EP-A- 0 485 683
- DE-A- 2 421 168
- GB-A- 1 419 270
- US-A- 4 319 067
- DATABASE WPI Section Ch, Week 7750 Derwent Publications Ltd., London, GB; Class E14, AN 77-89494y XP002008418 & SU-A-541 494 (UFA SYNTHETIC SPIRI) , 12.März 1977

## Beschreibung

Es ist bekannt, daß aromatische Kohlenwasserstoffe mit Olefinen oder Halogenkohlenwasserstoffen in Gegenwart von Friedel-Crafts-Katalysatoren zu Alkylaromaten umgesetzt werden. Katalysatoren können sein: Aluminiumchlorid, Aluminiumchlorid in Verbindung mit Chlorwasserstoff, Arylkomplexe des Aluminiumchlorids in Verbindung mit und ohne Chlorwasserstoff, Lewis-Säure wie z. B. BF₃, Antimonhalogenide oder Zinkhalogenide sowie deren Organyle und Aluminiumorganyle, Brønsted-Säuren wie Schwefelsäure, Phosphorsäure oder Fluorwasserstoff, im flüssigen Zustand oder auf poröse Trägermaterialien aufgebracht, sowie feste saure Katalysatoren wie Zeolithe oder andere amorphe oder kristalline Alumosilikate.

Eine ausführliche Zusammensetzung der möglichen Alkylierungskatalysatoren und -reaktionen geben G. A. Olah, 〈〈Friedel-Crafts and Related Reactions I, II〉〉 Interscience Publishers (1963, 1964) und W. Keim, M. Röper in Ullmanns's Encyclopedia of Industrial Chemistry, Vol. A1, S. 185 ff., Verlag Chemie, Weinheim 1991.

Das erfindungsgemäße Verfahren betrifft die Alkylierung von Aromaten wie Benzol, Naphthalin, Anthracen und deren substituierte Analoga wie z. B. Toluol, Ethylbenzol, Cumol, Xylol, Phenol, Anisol, Diphenyl, Diphenylmethane, Naphthole, vorzugsweise von Benzol mit Alkenen mit 3 oder mehr C-Atomen in der Flüssigphase zu Alkylbenzolen, vorzugsweise Propylen zu Cumol, an Aluminiumchlorid, Antimonhalogeniden oder Zinkhalogeniden, ohne und mit Chlorwasserstoff oder/auch ohne und mit Alkylhalogeniden wie z. B. Ethylchlorid, Propyl- oder Isopropylchlorid und analoge Alkylchloride. Die technischen Verfahren sind beschrieben beispielsweise in John J. McKetta, William A. Cunningham, Encyclopedia of Chemical Processing and Design, Vol. 14, 〈〈Cumene〉〉, S. 33 ff., Verlag Marcel Dekker, Inc., New York and Basel.

Bei der Alkylierung von Benzol mit Propylen entstehen in der Alkylierungsstufe neben Cumol auch mehrfach alkylierte Aromaten wie Diisopropyl-, Triisopropyl- oder Tetraisopropylbenzol, Indanderivate und Arylaromaten. Diese Art von Nebenreaktionen laufen auch ab, wenn Alkylierungskomponenten wie Ethylen, Butene, Pentene, Hexene oder die analogen Alkylhalogenide eingesetzt werden.

Diese mehrfach alkylierten Benzolderivate sieden bei höheren Temperaturen als die monoalkylierten Benzole und können in einer Destillationsstufe abgetrennt werden, die der destillativen Abtrennung von Cumol oder den entsprechenden anderen Monoalkylbenzolen nachgeschaltet ist.

Diese abgetrennten Neben- und Folgeprodukte lassen sich bekannterweise mit Benzol in Anwesenheit eines geeigneten Katalysators zu den Benzolderivaten, vorzugsweise zu Cumol, oder im Falle anderer Alkylierungskomponenten, zu den entsprechenden monoalkylierten Aromaten, wie z. B. Ethylenbenzol, Butyl- oder Isobutylbenzol, umalkylieren, was in der einschlägigen Literatur auch unter dem Begriff Transalkylierung behandelt wird.

In die Transalkylierung wird eine möglichst große Menge und auch ein breites Spektrum an Neben- und Folgeprodukten, soweit destillierbar, zur Umalkylierung gebracht, um eine hohe Ausbeute an Cumol oder monoalkylierten Produkten, bezogen auf den Gesamtprozeß, zu erreichen.

Die Transalkylierung kann gemäß FR-A 2 326 115 simultan im Alyklierungsreaktor erfolgen oder in separaten Transalkylierungsreaktoren, wie es in EP-A 0 046 678 beschrieben ist. Für die Transalkylierung lassen sich in der Flüssigphase als Katalysatoren Aluminiumchlorid, Antimonchlorid oder Zinkchlorid komplexiert und/oder mit Chlorwasserstoff oder auch als feste Katalysatoren Zeolithe verwenden, vorzugsweise protonenausgetauschte Y-Zeolithe, wie sie beispielsweise in EP-A 0 485 683 oder FR-A 2 662 557 genannt werden. DE-A 24 21 168 beschreibt ein Verfahren bei welchem vorzugsweise eine Katalysatorlösung aus Benzol, Diisopropylbenzol, Chlorwasserstoff und Aluminiumchlorid verwendet wird.

Ein Teilstrom oder bei diskontinuierlichen Verfahren eine Teilmenge der mehrfach alkylierten Benzolderivate wird gemäß US-B 4 319 067 zur Herstellung der Katalysator-Lösung verwendet. Aluminiumchlorid oder andere Metallhalogenide lösen sich in Gemischen von mehrfach alkylierten Aromaten. Eine solche Katalysatorlösung entsteht auch, wenn die Metalle mit mehrfach alkylierten Aromaten in Anwesenheit von Chlorwaserstoff oder Halogenkohlenwasserstoffen zusammengebracht werden.

Die Katalysatorlösung wird kontinuierlich oder diskontinuierlich der Alkylierung zugeführt. Sie läßt sich in gleicher Weise als Transalkylierungskatalysator verwenden und wird in den Transalkylierungsreaktor kontinuierlich oder diskontinuierlich eingebracht.

Überraschenderweise zeigte sich, daß die Herstellung der erfindungsgemäßen Katalysatorlösung mit einer Destillationsfraktion, die überwiegend di- und trialkylierte Benzole und weniger als 20 %, vorzugsweise weniger als 15 % Alkylbenzolderivate mit mehr als 3 Alkylgruppen am Benzol enthält, zu einer erheblichen Steigerung der Raum-Zeit-Ausbeute für die monoalkylierten Benzole führt und eine Senkung des Einsatzes von Katalysator, Chlorwasserstoff oder Halogenkohlenwasserstoff bezogen auf die produzierte Menge gestattet. Darüber hinaus ergeben sich bei der erfindungsgemäßen Herstellung des Katalysators verbesserte Produktqualitäten, insbesondere die Reduzierung von destillativ schwer abtrennbaren Nebenprodukten, z. B. n-Propylbenzol beim Cumol-Prozeß, und durch die Senkung des Katalysator-, Chlorwasserstoff- oder Halogenkohlenwasserstoff-Bedarfs deutlich verminderte Waschwassermengen in der Neutralisationsstufe sowie geringe Abwasserbelastungen, die in den Neutralisations- und Waschprozessen entstehen und als saure oder neutrale Fracht mit Wasser ausgeschleust werden.

Besonders gute Ergebnisse werden erzielt, wenn das zur Komplexierung und/oder Auflösung des Katalysators erforderliche Gemisch aus Benzol und alkylierten Benzolderivaten besteht, die mehr als 60 %, vorzugsweise mehr als 80 %, dialkylierte und/oder trialkylierte Benzolderivate enthalten. Die alkylierten Benzolderivate sollten dabei weniger als 20 %, vorzugsweise weniger als 15 %, Alkylbenzolderivate mit mehr als 3 Alkylgruppen am Benzol enthalten.

Die Erfindung betrifft gleichermaßen die Transalkylierungsreaktionen, die bei einem Verzicht auf höher alkylierte Benzolderivate und Arylbenzole als Einsatzstoffe für die Katalysatorlösung und in den Umalkylierungsprozeß ebenfalls höhere Raum-Zeit-Ausbeute für die monoalkylierten Benzole erreichen lassen. Darüber hinaus wird bei festen Transalkylierungskatalysatoren, vorzugsweise bei zeolithischen Katalysatoren, die Bildung von desaktivierenden Ablagerungen reduziert, so daß ihre Aktivität länger auf einem ökonomisch vertretbarem Niveau erhalten bleibt. Die Transalkylierung wird heterogen-katalytisch an sauren Zeolithen im gefluteten Festbett, an suspendierten sauren Zeolithen oder im Rieselbett durchgeführt. Diese höher alkylierten Produkte führen nur zu einer geringfügigen Erhöhung des Rückstandsanfalls, der umweltschonend z. B. zu Synthesegas vergast werden kann.

In der Literatur und den zitierten Patenten wird auf die Bedeutung und Auswirkung von hochalkylierten Benzolderivaten nicht eingegangen. In der Regel werden verallgemeinernd Polyalkylbenzole oder alkylierte Öle in die Transalkylierung geführt. Gemäß JP 04 066 543 werden Polyisopropylbenzole und Trimethylindan erhitzt, um Produkte höheren Molekulargewichts zu erhalten; auf diese Weise muß man einen teilweisen Verbrauch von dialkylierten Benzolderivaten und somit Ausbeuteverluste in Kauf nehmen.

Bei dem erfindungsgemäßen Verfahren zur Alkylierung und Transalkylierung von Aromaten mit einem homogenen Katalysator werden die höher alkylierten Alkylierungsprodukte abgetrennt und sowohl zur Herstellung eines homogenen Alkylierungs- und Transalkylierungskatalysators eingesetzt als auch einer homogen- oder heterogenkatalytischen Transalkylierung unterzogen, wobei das zur Komplexierung und/oder Auflösung des Katalysators erforderliche Gemisch aus Benzol und alkylierten Benzolderivaten, welche mehr als 60 %, vorzugsweise mehr als 80 %, dialkylierte und/oder trialkylierte Benzolderivate enthalten, besteht.

Die Alkylierung von Benzol und anderen Aromaten sowie deren Substitutionsprodukte mit Ethylen, Propylen, Buten oder mit halogenierten Kohlenwasserstoffen erfolgt in der Flüssigphase an gelösten oder dispergierten Kontakten bei 60 bis 180 °C und Drücken von 1 bis 10 bar, die Alkylierung von Benzol mit Propylen wird vorzugsweise zwischen 80 und 130 °C bei Drücken bis zu 4 bar durchgeführt. Die Molverhältnisse von Alkylierungskomponenten zu Aromaten liegen zwischen 1 : 2 bis 1 : 5, vorzugsweise zwischen 1 : 2,5 bis 1 : 3,5. An die Reaktionskomponenten sind hohe Anforderungen bezuglich Reinheit zu stellen. Auch in sehr geringen Konzentrationen stören Wasser, Aldehyde, sonstige ungesättigte oder halogenierte Kohlenwasserstoffe, anorganische und organische Säuren und Basen sowie Alkohole.

Als Reaktoren lassen sich auf verschiedene Weisen begaste Rührkessel oder geflutete Reaktionskolonnen mit und ohne Rühraggregate verwenden. Auch eine flüssige Dosierung der Alkylierungskomponenten ist möglich, was im Falle des Ethylens, Propylens, Methylchlorids und Butens entsprechend höhere Drücke erforderlich macht.

Die Alkylierungsprozesse können sowohl kontinuierlich als auch diskontinuierlich geführt werden. Im Falle des kontinuierlichen Betriebs liegen die mittleren Verweilzeiten der Flüssigphase bei 30 Minuten bis 2 Stunden.

Um den komplexen Katalysator in Lösung zu halten, dürfen die Konzentrationen nicht beliebig hoch gewählt werden. Im Falle AlCl₃ komplexiert mit Polyalkylbenzolen, liegt die Löslichkeitsgrenze in Benzol bei ca. 5 % (berechnet auf Aluminium).

Die Herstellung der Katalysatormischung erfolgt separat bei ca. 80 °C, unter Eingasen von trocknem Chlorwasserstoff und Rühren. Das Verhältnis von alkylierten Aromaten als Komplexbilder zu unsubstituierten Aromaten liegt zwischen 2 : 1 und 10 : 1, im Falle Benzol vorzugsweise 3 : 1.

Dieses Gemisch wird sowohl in der Alkylierung als auch in der Transalkylierung in gleichen Konzentrationen (≤ 4 % Aluminium) als Katalysator eingesetzt.

Die Transalkylierungstemperaturen für homogene Prozeßführung liegen ebenfalls zwischen 80 und 180 °C und bei Drücken bis zu 10 bar, vorzugsweise zwischen 100 und 130 °C und bei 2 bis 3 bar.

Als Katalysatoren können Aluminiumchlorid, Aluminiumchlorid in Verbindung mit Chlorwasserstoff, Arylkomplexe des Aluminiumchlorids mit und ohne Chlorwasserstoff, Lewis-Säuren, wie z. B. BF₃, Antimonhalogenide oder Zinkhalogenide hergestellt aus den Metallen sowie deren Organyle und Aluminiumorganyle verwendet werden. Vorzugsweise wird ein Aluminiumchlorid verwendet, das direkt aus Aluminium mit Chlorwasserstoff entsteht und unmittelbar durch alkylierte Aromaten komplexiert wird. Die Katalysatoren werden in, wie in der Literatur beschrieben, üblichen Mengen eingesetzt.

### Beispiele:

1. Katalysator
1.1 Für einen kontinuierlich betriebenen, begasten Rührkesselreaktor wird eine Katalysatorlösung für die Alkylierung von Benzol zu Cumol vorbereitet mit
   - 1 900 g: Benzol
   - 130 g: Aluminiumgrieß
   - 600 g: Polyalkylbenzol
   Die Zusammensetzung des Polyalkylbenzols entspricht der üblichen Fahrweise
   - 60 %: Diisopropylenbenzole
   - 8 %: Trimethylindan
   - 3 %: Triisopropylbenzol
   - 29 %: höher alkylierte Produkte
   Durch die Suspension wurde unter Rühren so lange Chlorwasserstoff hindurchgeleitet, bis kein metallisches Aluminium mehr sichtbar war.
1.2 In einem weiteren Ansatz wurde wie unter 1.1 verfahren, mit dem Unterschied, daß ein Polyalkylbenzol eingesetzt wurde mit
   - 80 %: Diisopropylenbenzolen
   - 3 %: Trimethylindan
   - 3 %: Triisopropylbenzolen
   - 14 %: höher alkylierte Produkten
1.3 In einem dritten Katalysatoransatz, der ebenfalls wie unter 1.1 durchgeführt wurde, wurde statt Polyalkylbenzol ein Gemisch aus
   - 97 %: Diisopropylenbenzolen
   - 3 %: Triisopropylbenzolen
   verwendet.

2. Alkylierung zum Cumol
Für die katalytischen Alkylierungen steht ein kontinuierlich betreibbarer, begasungsfähiger Rührkessel von 90 l zur Verfügung, der sowohl beheizbar als auch kühlbar ist und bis zu Drücken von 5 bar sowie Temperaturen bis zu ca. 150 °C unter stark sauren Bedingungen belastbar ist. Unter Reaktionsbedingungen wurde immer so verfahren, daß das eingegaste Propylen vollständig umgesetzt wurde.
Folgende Ergebnisse wurden mit den Katalysatoren 1.1 bis 1.3 erhalten (Druck = 2 bar, Temperatur = 120 °C):

| Kat. | Einsätze | | | | Cumol im Austrag [%] | Raum-Zeit-Ausbeute [kg/h · l] |
|---|---|---|---|---|---|---|
| | Propen [kg/h] | Benzol [kg/h] | HClg [lₙ/h] | Kat. [cm³/h] | | |
| 1.1 | 6,25 | 32 | 60 | 60 | 32,5 | 0,136 |
| 1.2 | 8,5 | 33 | 45 | 45 | 36,0 | 0,166 |
| 1.3 | 9,2 | 33 | 40 | 40 | 37,5 | 0,176 |

Der Propen-Einsatz war jeweils durch die Bedingungen begrenzt, daß es zu keinen Olefin-Durchbruch kommen durfte. Die Zusammensetzung des Katalysators bestimmt somit die Raum-Zeit-Ausbeute, die im Vergleich zu dem normalen Katalysator 1.1 um bis zu 29 % gesteigert werden kann.
3. Transalkylierung zum Cumol
Die Transalkylierung wurde ebenfalls in dem 90 l-Rührkessel durchgeführt (Druck = 2 bar, Temperatur = 120 °C). Die Einsätze an Polyalkylbenzolen der Zusammensetzungen gemäß 1.1 bis 1.3 betrugen jeweils 11,5 kg/h, die Katalysatordosierung lag bei 40 cm³/h und der Benzoleinsatz war auf 30 kg/h festgesetzt.

| Kat. | Polyalkylbenzol-Einsatz gemäß | Cumol im Austrag [%] | Raum-Zeit-Ausbeute [kg/h·l] |
|---|---|---|---|
| 1.1 | 1.1 | 13,7 | 0,063 |
| 1.1 | 1.3 | 20,2 | 0,093 |
| 1.2 | 1.1 | 16,8 | 0,077 |
| 1.2 | 1.2 | 20,2 | 0,093 |
| 1.2 | 1.3 | 21,5 | 0,099 |
| 1.3 | 1.3 | 22,2 | 0,102 |

Die Beispiele machen deutlich, daß durch höher alkylierte Benzolderivate sowohl die Wirkung des Katalysators als auch die Transalkylierung selbst nachteilig beeinflußt werden.
4. Übertragung in den technischen Prozeß
Die Ergebnisse der halbtechnischen Versuche gemäß Beispiel 2 und 3 wurden auf den technischen Prozeß übertragen. Dazu wurde die Destillation der Polyalkylbenzole so eingestellt, daß das Destillat eine Zusammensetzung von mindestens 80 % Diisopropylbenzol gemäß Beispiel 1.2 hatte.
Der Katalysator wurde mit diesem Destillat hergestellt und die Transalkylierung mit diesem Polyalkylbenzol-Gemisch beschickt. Hierdurch konnte die Raum-Zeit-Ausbeute um 25 % gesteigert werden. Zusätzlich ließen sich reduzieren

| | |
|---|---|
| die Katalysatormenge um | 30 % |
| der Chlorwasserstoffeinsatz um | 30 % |
| die Waschwassermenge um | 40 % |
| die Salzproduktion in der Waschstufe um | 40 % |

Die Bildung von unerwünschtem n-Propylbenzol, das sich in der Anlage von Cumol destillativ nicht abtrennen läßt, wurde soweit reduziert, daß sein Gehalt im Cumol um 400 ppm zurückging.
Die veränderte Fahrweise führte zu einer um 3 t/h erhöhten Produktion von Rückständen, die gegenüber der gestiegenen Produktivität der Anlage ohne wirtschaftliche Bedeutung sind und umweltfreundlich zu Synthesegas vergast werden.
5. Alkylierungen und Transalkylierungen mit Buten-2 und Benzol
Die Technikumsversuche im o. g. Reaktor mit einem Katalysator analog 1.1 und Alkylierungsbedingungen gemäß 2. führten gegenüber der Alkylierung zum Cumol zu etwa 20 % niedrigeren Raum-Zeit-Ausbeuten. Aber auch hierbei zeigte sich, daß höher alkylierte Benzolderivate die Wirkung des homogenen Katalysators reduzieren. Die Reduzierung von höher alkylierten Benzolderivaten auf 10 % führten zu einer Steigerung der Raum-Zeit-Ausbeute um 20 %.
6. Transalkylierung von Polyalkylbenzolen zu Cumol an einem heterogenen Katalysator in einem Festbettreaktor
In einem kontinuierlich betreibbaren Festbettreaktor mit 5 l-Inhalt und einem Durchmesser von 80 mm wurde bei 35 bar und 270 °C die Transalkylierung an einem protonenausgetauschten NaY-Zeolith durchgeführt. Die Pellets hatten einen Durchmesser von 3 bis 5 mm und eine BET-Oberfläche von 80 m²/g. Dem Reaktor wurden zugeführt 2 kg/h Benzol und 0,5 kg/h Polyalkylbenzole entsprechend der Zusammensetzung in 1.1 bis 1.3. Dabei war zu beobachten, daß die Raum-Zeit-Ausbeuten mit der Versuchsdauer sich änderten:

| Polyalkylbenzole entsprechend | Raum-Zeit-Ausbeute an Cumol [kg/h·l] | | |
|---|---|---|---|
| | 1. Tag | 10. Tag | 30. Tag |
| 1.1 | 0,106 | 0,080 | 0,032 |
| 1.2 | 0,124 | 0,108 | 0,102 |
| 1.3 | 0,143 | 0,122 | 0,118 |

Diese Versuchsergebnisse zeigen deutlich, daß die Einspeisung eines Polyalkylbenzolgemisches mit größeren Anteilen von höherer alkylierten Benzolderivaten zu einer Aktivitätsminderung und schnelleren Desaktivierung des heterogenen Transalkylierungskatalysators führen.

## Patentansprüche

1. Verfahren zur Alkylierung und Transalkylierung von Aromaten mit einem homogenen Katalysator,
dadurch gekennzeichnet,
daß die höher alkylierten Alkylierungsprodukte abgetrennt und sowohl zur Herstellung eines homogenen Alkylierungs- und Transalkylierungskatalysators verwendet als auch einer homogen- oder heterogenkatalytischen Transalkylierung unterzogen werden und daß das zur Komplexierung und/oder Auflösung des Katalysators erforderliche Gemisch aus Benzol und alkylierten Benzolderivaten, welche mehr als 60 %, vorzugsweise mehr als 80 %, dialkylierte und/oder trialkylierte Berzolderivate enthalten, besteht.

2. Verfahren gemäß Anspruch 1,
dadurch gekennzeichnet,
daß die zur Komplexierung und/oder Auflösung des Katalysators erforderlichen alkylierten Berzolderivate weniger als 20 %, vorzugsweise weniger als 15 %, Alkylbenzolderivate mit mehr als 3 Alkylgruppen am Benzol enthalten.

3. Verfahren nach Anspruch 1,
dadurch gekennzeichnet,
daß Benzol mit Propylen alkyliert wird.

4. Verfahren nach zumindest einem der vorherigen Ansprüche,
dadurch gekennzeichnet,
daß als homogener Katalysator Aluminium-, Antimon- und/oder Zinkchloride, hergestellt aus den Metallen, mit oder ohne Chlorwasserstoff eingesetzt werden.

5. Verfahren nach zumindest einem der vorherigen Ansprüche,
dadurch gekennzeichnet,
daß das Verhältnis von Alkylierungskomponenten zu Aromaten zwischen 1 : 2 bis 1 : 5 liegt.

6. Verfahren nach zumindest einem der vorherigen Ansprüche,
dadurch gekennzeichnet,
daß als Transalkylierungskatalysatoren Zeolithe oder andere saure, amorphe oder kristalline Alumosilikate eingesetzt werden.

7. Verfahren nach zumindest einem der vorherigen Ansprüche,
dadurch gekennzeichnet,
daß die Alkylierung in der flüssigen Phase mit flüssig oder gasförmig eingespeisten Alkylierungskomponenten stattfindet.

8. Verfahren nach zumindest einem der vorherigen Ansprüche,
dadurch gekennzeichnet,
daß die Transalkylierung heterogen-katalytisch an sauren Zeolithen im gefluteten Festbett, an suspendierten sauren Zeolithen oder im Rieselbett stattfindet.

## Claims

1. A process for the alkylation and transalkylation of aromatics using a homogeneous catalyst, characterized in that the relatively highly alkylated alkylation products are separated off and are not only used for preparing a homogeneous alkylation and transalkylation catalyst but also subjected to a homogeneously or heterogeneously catalysed transalkylation and in that the mixture required for complexation and/or dissolution of the catalyst comprises benzene and alkylated benzene derivatives which contain more than 60%, preferably more than 80%, of dialkylated and/or trialkylated benzene derivatives.

2. A process according to claim 1, characterized in that the alkylated benzene derivatives required for complexation and/or dissolution of the catalyst contain less than 20%, preferably less than 15%, of alkylbenzene derivatives having more than 3 alkyl groups on the benzene.

3. A process according to claim 1, characterized in that benzene is alkylated with propylene.

4. A process according to at least one of the preceding claims, characterized in that aluminium chloride, antimony chloride and/or zinc chloride, prepared from the metals, are used with or without hydrogen chloride as homogeneous catalyst.

5. A process according to at least one of the preceding claims, characterized in that the ratio of alkylation components to aromatics is from 1:2 to 1:5.

6. A process according to at least one of the preceding claims, characterized in that zeolites or other acidic, amorphous or crystalline aluminosilicates are used as transalkylation catalysts.

7. A process according to at least one of the preceding claims, characterized in that the alkylation takes place in the liquid phase using alkylation components fed in in liquid or gaseous form.

8. A process according to at least one of the preceding claims, characterized in that the transalkylation takes place heterogeneously catalyzed over acid zeolites in a flooded fixed bed, over suspended acid zeolites or in a trickle bed.

## Revendications

1. Procédé d'alkylation et de transalkylation d'aromates avec un catalyseur homogène, caractérisé en ce qu'
- on sépare les produits d'alkylation alkylés supérieurs et on les utilise tant pour la fabrication d'un catalyseur d'alkylation et de transalkylation homogène que pour les soumettre aussi à une transalkylation homogéno- ou hétérogéno-catalytique, et
- le mélange nécessaire pour la complexité et/ou la dissolution du catalyseur se compose de benzène et de dérivés de benzène alkylés qui contiennent plus de 60%, de préférence plus de 80 %, de dérivés de benzène dialkylés et/ou trialkylés.

2. Procédé selon la revendication 1,
caractérisé en ce que
les dérivés de benzène alkylés nécessaires pour la complexité et/ou la dissolution du catalyseur contiennent moins de 20 %, de préférence moins de 15 %, de dérivés d'alkylbenzène comportant plus de trois groupes alkyle sur le noyau benzène.

3. Procédé selon la revendication 1,
caractérisé en ce qu'
on alkyle le benzène avec du propylène.

4. Procédé selon au moins une des revendications précédentes, caractérisé en ce qu'on utilise comme catalyseur homogène les chlorures d'aluminium, d'antimoine et/ou de zinc, obtenus à partir des métaux, avec ou sans acide chlorhydrique.

5. Procédé selon au moins une des revendications précédentes,
caractérisé en ce que
le rapport des composants d'alkylation aux composés aromatiques se situe entre 1:2 et 1:5.

6. Procédé selon au moins une des revendications précédentes,
caractérisé en ce qu'
on utilise comme catalyseurs de transalkylation des zéolithes ou d'autres aluminosilicates acides, amorphes ou cristallins.

7. Procédé selon au moins une des revendications précédentes,
caractérisé en ce que
l'alkylation se déroule en phase liquide avec des composants d'alkylation liquides ou gazeux accumulés.

8. Procédé selon au moins une des revendications précédentes,
caractérisé en ce que
la transalkylation s'effectue de façon hétérogénocatalytique dans des zéolithes acides dans un lit fixe inondé, sur des zéolithes acides en suspension ou dans un lit à ruissellement.
